(19) Europäisches Patentamt
European Patent Office
Office européen des brevets

(11) Veröffentlichungsnummer: **0 081 679**
**B1**

(12) **EUROPÄISCHE PATENTSCHRIFT**

(45) Veröffentlichungstag der Patentschrift: **29.01.86**

(51) Int. Cl.⁴: **C 07 D 233/68**, A 01 N 43/50

(21) Anmeldenummer: **82110343.9**

(22) Anmeldetag: **10.11.82**

(54) **Substituierte Benzylimidazoliumsalze und diese enthaltende Mikrobizide.**

(30) Priorität: **20.11.81 DE 3145928**
**20.11.81 DE 3145927**

(43) Veröffentlichungstag der Anmeldung:
**22.06.83 Patentblatt 83/25**

(45) Bekanntmachung des Hinweises auf die Patenterteilung:
**29.01.86 Patentblatt 86/5**

(84) Benannte Vertragsstaaten:
**AT BE CH DE FR GB IT LI NL SE**

(56) Entgegenhaltungen:
**EP - A - 0 005 528**
**EP - A - 0 028 834**
**DE - A - 2 647 940**
**DE - B - 1 216 487**

(73) Patentinhaber: **BASF Aktiengesellschaft,**
**Carl-Bosch-Strasse 38, D-6700 Ludwigshafen (DE)**

(72) Erfinder: **Dockner, Toni, Dr., Grossgasse 6,**
**D-6701 Meckenheim (DE)**
Erfinder: **Pommer, Ernst-Heinrich, Dr., Berliner Platz 7,**
**D-6703 Limburgerhof (DE)**
Erfinder: **Wetzler, Matthias, Liebermannstrasse 7,**
**D-6700 Ludwigshafen (DE)**

**EP 0 081 679 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue, wertvolle substituierte Benzylimidazoliumsalze, Mikrobizide, die diese Verbindungen enthalten und Verfahren zur Bekämpfung von Mikroben mit diesen Verbindungen.

Es ist bekannt, das N-Dodecyl-N,N-dimethyl-N-benzyl-ammoniumchlorid (Wallhäusser: Sterilisation-Desinfektion-Konservierung-Chemotherapie, Georg Thieme Verlag, Stuttgart, 1967, S. 222) oder das 1,3-Bis-(p-isobutylbenzyl)-imidazoliumchlorid (DE-PS 1 216 487) zur Bekämpfung von Mikroben zu verwenden.

Es wurde gefunden, dass substituierte Benzylimidazole der Formel

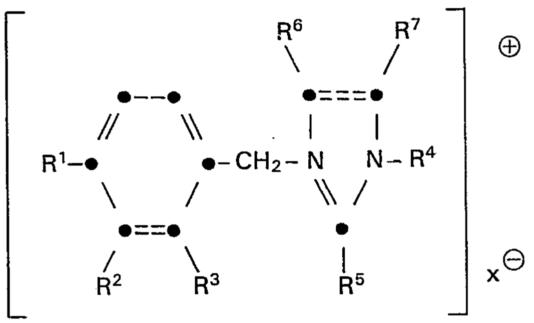

in der $R^1$, $R^2$ und $R^3$ gleich oder verschieden sind und Wasserstoff, einen $C_1$-$C_4$-Alkylrest oder Halogen, $R^4$ einen Alkylrest mit 8 bis 14 Kohlenstoffatomen, Benzyl, halogen- bzw. alkylsubstituiertes Benzyl, $R^5$ Wasserstoff oder einen Alkylrest mit 1 bis 8 Kohlenstoffatomen, Phenyl, Benzyl, $R^6$ Alkyl mit 1 bis 4 C-Atomen oder Halogen, $R^7$ Alkyl mit 1 bis 4 C-Atomen oder Halogen und X einen Säurerest, insbesondere von Halogenwasserstoffsäuren, bedeuten, eine sehr gute Wirksamkeit gegenüber Bakterien, Pilzen und Algen haben.

Ein $C_1$-$C_4$-Alkylrest ist beispielsweise Methyl, Ethyl, Butyl, t-Butyl. $R^4$ ist beispielsweise Octyl, Decyl, Dodecyl, Benzyl, 2-Methyl-4-chlor-benzyl, 2-Methyl-3,4-dichlor-benzyl, 2-Methyl-3-chlor-benzyl, Halogen ist beispielsweise Chlor, Brom oder Jod.

Ein Alkylrest mit 1 bis 8 Kohlenstoffatomen ist beispielsweise Methyl, Ethyl, Hexyl, Octyl.

Ein Säurerest ist beispielsweise der Rest einer anorganischen oder organischen Säure, z.B. Salzsäure, Bromwasserstoffsäure, Dodecylbenzolsulfonsäure, Essigsäure, Salicylsäure, Benzoesäure. Bevorzugt werden die Salze von Halogenwasserstoffsäuren.

Die neuen Verbindungen werden beispielsweise hergestellt, indem man eine Verbindung der Formel

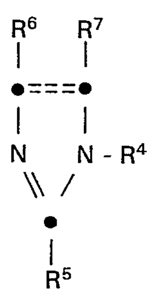

in der $R^4$, $R^5$, $R^6$, $R^7$ die oben genannten Bedeutungen haben in der Schmelze mit einer Verbindung der Formel

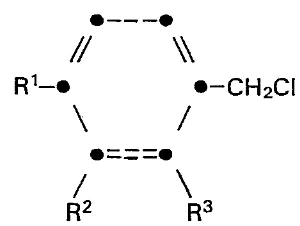

in der $R^1$, $R^2$, $R^3$ die oben genannten Bedeutungen haben, umsetzt. Es werden beispielsweise stöchiometrische Mengen der Ausgangssubstanzen verwendet. Die Umsetzung erfolgt beispielsweise ohne Lösungsmittel in der Schmelze des Imidazols. Die Umsetzung erfolgt je nach Schmelztemperatur bei etwa 50 bis 130°C.

*Beispiel 1*

Herstellung von 1-Dodecyl-3-(2-methyl-4-chlor-benzyl)-4-methyl-5-iodimidazoliumchlorid

37,9 g (0,1 Mol) 1-Dodecyl-4-methyl-5-iodimidazol wurden vorgelegt und auf 100°C erhitzt und

17,7 g (0,1 Mol) 4-Chlor-orthoxylylchlorid zugetropft. Das Reaktionsgemisch wurde bei 120 bis 130°C nachgerührt. Die entstandene Verbindung (Verbindung Nr. 7) entspricht folgender Formel

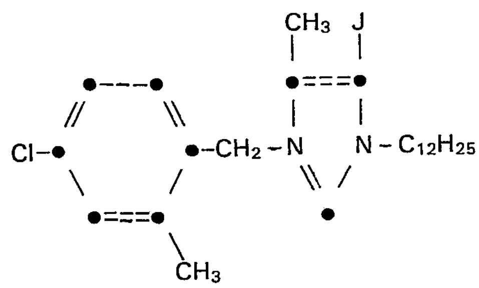

Analyse:

| | errechnet: | gefunden: |
|---|---|---|
| C | 51,98 | 52,7 |
| H | 6,67 | 7,1 |
| N | 5,05 | 5,2 |
| Cl | 12,81 | 12,1 |
| J | 23,46 | 22,7 |

*Beispiel 2*

Herstellung von 1-Decyl-3-(2-methyl-3-chlor-benzyl)-4-methyl-5-bromimidazoliumchlorid

60,2 g (0,2 Mol) 1-Decyl-4-methyl-5-bromimidazol wurden auf 100°C erhitzt und

35,4 g (0,2 Mol) 3-Chlor-orthoxylylchlorid zugetropft. Das Reaktionsgemisch wurde 30 Minuten bei 120 bis 130°C nachgerührt.

Die flüssige Verbindung (Verbindung Nr. 8) entspricht folgender Analyse:

Analyse:

| | errechnet: | gefunden: |
|---|---|---|
| C | 55,46 | 56,1 |
| H | 6,93 | 7,2 |
| N | 5,88 | 5,9 |
| Cl | 14,91 | 14,1 |
| Br | 16,81 | 15,9 |

*Beispiel 3*

Herstellung von 1,3-Di-(2-methyl-4-chlor-benzyl)--4-methyl-5-chlorimidazoliumchlorid

255 g (1 Mol) 1-(2-methyl-4-chlor-benzyl)-4--methyl-5-chlorimidazol wurden auf 80°C erhitzt und

175 g (1 Mol) 4-Chlor-orthoxylylchlorid (2-Methyl-4-chlor-benzylchlorid) zugetropft und anschliessend ca. 1 Stunde bei dieser Temperatur nachgerührt.

Es entstanden 430 g einer Verbindung (Verbindung Nr. 6) folgender Zusammensetzung:

| | gefunden: | errechnet: |
|---|---|---|
| C | 56,5% | 55,81% |
| H | 5,0% | 4,65% |
| N | 6,8% | 6,51% |
| Cl | 32,1% | 33,0% |

*Beispiel 4*

Herstellung von 1-Decyl-3-(2-methyl-4-chlor--benzyl)-4-methyl-5-chlorimidazoliumchlorid

25,6 g (0,1 Mol) 1-Decyl-4-methyl-5-chlorimidazol wurden auf 100°C erhitzt und dazu

17,7 g (0,1 Mol) 4-Chlor-o-xylylchlorid zugetropft und 30 Minuten bei 125°C nachgerührt.

Es entstanden 43 g der Verbindung mit dem Schmelzpunkt 99°C (Verbindung Nr. 3).

*Beispiel 5*

Herstellung von 1-Dodecyl-3-(2-methyl-3-chlor--benzyl)-4-methyl-5-chlorimidazoliumchlorid

14,2 g (0,05 Mol) 1-Dodecyl-4-methyl-5-chlorimidazol wurden auf 95°C erhitzt und

8,4 g (0,05 Mol) 3-Chlor-o-xylylchlorid zugetropft und 30 Minuten bei 95°C nachgerührt.

Es entstanden 22,6 g des Endproduktes mit einem Schmelzpunkt von 112°C (Verbindung Nr. 5). Die Ausbeuten waren quantitativ.

*Beispiel 6*

Herstellung von 1-Decyl-3-benzyl-4-methyl-5--chlor-imidazoliumchlorid

25,5 g (0,1 Mol) 1-Decyl-4-methyl-5-chlorimidazol wurden auf 100°C erhitzt und innerhalb 10 Minuten

12,6 g (0,1 Mol) Benzylchlorid zugetropft und anschliessend 30 Minuten bei 125°C nachgerührt.

Es wurden 38 g (0,1 Mol) des Endproduktes (Verbindung Nr. 1) gewonnen. Die Ausbeuten waren quantitativ.

Entsprechend wurden diejenigen in der folgenden Tabelle angegebenen Verbindungen hergestellt, deren Schmelzpunkte (Fp) angegeben sind.

Ihre Struktur wurde durch Elementaranalyse gesichert. Die Verbindungen, bei denen keine Angaben zur physikalischen Chemie gemacht sind, können auf die gleiche Art und Weise wie bei den tatsächlich hergestellten Verbindungen erhalten werden; es kann erwartet werden, dass sie aufgrund ihrer ähnlichen Konstitution ähnliche Wirkungen wie die näher untersuchten Verbindungen haben.

| Wirkstoff Nr. | $R^1$ | $R^2$ | $R^3$ | $R^4$ | $R^5$ | $R^6$ | $R^7$ | X | Fp. |
|---|---|---|---|---|---|---|---|---|---|
| 1 | H | H | H | $C_{10}H_{21}$ | H | $CH_3$ | Cl | Cl | |
| 2 | H | Cl | $CH_3$ | $C_{10}H_{21}$ | H | $CH_3$ | Cl | Cl | 125°C |
| 3 | Cl | H | $CH_3$ | $C_{10}H_{21}$ | H | $CH_3$ | Cl | Cl | 99°C |
| 4 | Cl | H | $CH_3$ | $C_{12}H_{25}$ | H | $CH_3$ | Cl | Cl | |
| 5 | H | Cl | $CH_3$ | $C_{12}H_{25}$ | H | $CH_3$ | Cl | Cl | 112°C |
| 6 | Cl | H | $CH_3$ | 2-$CH_3$-4-Cl-Benzyl | H | $CH_3$ | Cl | Cl | |
| 7 | Cl | H | $CH_3$ | $C_{12}H_{25}$ | H | $CH_3$ | J | Cl | |
| 8 | Cl | H | $CH_3$ | $C_{10}H_{21}$ | H | $CH_3$ | Br | Cl | |
| 9 | H | H | Cl | $C_{10}H_{21}$ | H | $CH_3$ | Br | Cl | |
| 10 | Cl | H | H | $C_{10}H_{21}$ | H | $CH_3$ | Br | Cl | |
| 11 | H | H | Cl | $C_{12}H_{25}$ | H | $CH_3$ | J | Cl | |
| 12 | Cl | H | H | $C_{12}H_{25}$ | H | $CH_3$ | J | Cl | |

Die Herstellung der Ausgangsprodukte der Formel

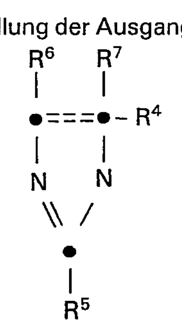

in der $R^4$, $R^5$, $R^6$, $R^7$ die oben genannten Bedeutungen haben, erfolgt in der Weise, dass man eine Verbindung der Formel

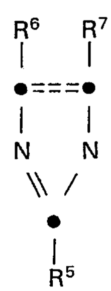

in der $R^5$, $R^6$, $R^7$ die oben genannten Bedeutungen haben, in einem Lösungsmittel, z.B. Dimethylformamid, bei Temperaturen von 100 bis 150°C während 1 bis 20 Stunden mit $NaOCH_3$ umsetzt, das $CH_3OH$

abdestilliert, das so erhaltene Produkt mit einer Verbindung der Formel $R^4$-Cl umsetzt, wobei $R^4$ die oben genannten Bedeutungen hat, das NaCl von der Reaktionsmischung abtrennt und das Endprodukt aus dem Lösungsmittel isoliert.

Man erhält auf diese Weise beispielsweise die folgenden Verbindungen:

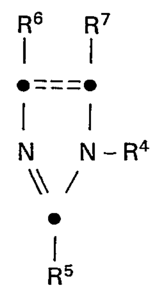

| $R^4$ | $R^5$ | $R^6$ | $R^7$ | Siedebereich |
|---|---|---|---|---|
| $C_{10}H_{21}$ | H | $CH_3$ | Cl | 178-211 °C/8 mbar |
| $C_{12}H_{23}$ | H | $CH_3$ | Cl | 175-221 °C/8 mbar |

Die Herstellung des Ausgangsproduktes der Formel

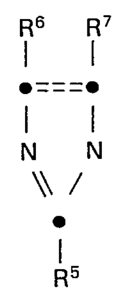

in der $R^5$, $R^6$, $R^7$ die oben genannten Bedeutungen haben, erfolgt in der Weise, dass man z.B. 4-Methylimidazol mit Chlorgas im ultravioletten Licht bei 20 bis 50 °C in einem Lösungsmittel, z.B. Tetrachlorkohlenstoff oder Monochlorbenzol, in 2 bis 10 Stunden zum Hydrochlorid des 4-Methyl-5-chlor-imidazols umsetzt und 4-Methyl-5-chlorimidazol aus seinem Hydrochlorid mit Alkali, z.B. $NaHCO_3$ freisetzt.

Die Verbindung schmilzt bei 130 bis 132 °C.

Die neuen Verbindungen eignen sich zur Bekämpfung von Mikroorganismen (Mikroben) z.B. zum Schutz von Wasser gegen Algenbefall, zur Bekämpfung von schleimbildenden Mikroorganismen in Rückkühlwerken, in der Papierindustrie oder in Luftbefeuchtungsanlagen. Ferner können sie als Desinfektionsmittel oder Konservierungsmittel für den technischen Bereich sowie als Bakterizide im Pflanzenschutz eingesetzt werden.

Folgende Mikroorganismen lassen sich beispielsweise mit den neuen Wirkstoffen bekämpfen: Staphylococcus aureus, Escherichia coli, Klebsiella pneumoniae, Citrobacter freundii, Proteus vulgaris, Pseudomonas aeruginosa, Pseudomonas fluorescens, Xanthomonas vesicatoria, Xanthomonas malvacearum, Erwinia carotovora, Erwinia amylovora, Desulfovibrio desulfuricans, Streptoverticillium rubrireticuli, Aspergillus niger, Aspergillus versicolor, Penicillium funiculosum, Paecilomyces variotii, Trichoderma viride, Chaetomium globosum, Candida albicans, Geotrichum candidans, Monilia sitophila, Scenedesmus quadricauda, Chlorella vulgaris, Nostoc muscorum.

Die neuen Verbindungen sind in Wasser leicht löslich. Sie werden daher bevorzugt in Form ihrer wässrigen Lösung angewendet. Konzentrierte Zubereitungen können auch mit organischen Lösungsmitteln, z.B. Ethanol, hergestellt werden.

Die üblichen Anwendungskonzentrationen betragen 0,01 bis 1 % Wirkstoff, bezogen auf das Gewicht des zu schützenden Materials; beim Einsatz zur Wasserbehandlung bei der Erdölförderung, in Schwimmbädern, Rückkühlwerken, Luftbefeuchtungsanlagen, Blumenfrischhaltemittel oder in der Papierindustrie sind Wirkstoffmengen von 5 bis 100 ppm ausreichend, um eine Mikroorganismenentwicklung zu unterdrücken. Gebrauchsfertige Desinfektionsmittellösungen enthalten 0,5 bis 10% (Gewichtsprozent) Wirkstoff.

Im Pflanzenschutz betragen die Aufwandmengen 0,1 bis 5 kg Wirkstoff je ha. Zubereitungen mit organischen Lösungsmitteln können 1 bis 60% Wirkstoff enthalten.

Die Wirkstoffe können auch mit anderen bekannten Mikrobiziden gemischt werden. In vielen Fällen erhält man dabei eine Vergrösserung des mikrobiziden Wirkungsspektrums, bei einer Anzahl dieser Mischungen treten synergistische Effekte auf, d.h. die mikrobizide Wirksamkeit des Kombinationsproduktes ist grösser als die Summe der addierten Wirksamkeit der Einzelkomponenten.

Wirkstoffe, die mit den erfindungsgemässen Benzylimidazolen kombiniert werden können, sind beispielsweise:

2-(Thiocyanomethylthio)-benzthiazol
1-[2-(2,4-Dichlorphenyl)-2-(propenyl-oxy)-ethyl]-1H-imidazol
2,4,5,6-Tetrachlor-isophthalodinitril
Methylenbisthiocyanat
Tributylzinnoxid
Mercaptobenzthiazol
Benzisothiazolon und seine Alkalisalze
Alkaliverbindungen des N'-Hydroxy-N-cyclohexyl-diazeniumoxids
2-(Methoxy-carbonylamino)-benzimidazol
2-Methyl-3-oxo-5-chlor-thiazolin-3-on
Trihydroxymethyl-nitro-methan
Glutardialdehyd
Chloracetamid
quaternäre Ammoniumverbindungen.

In nachstehenden Versuchen wurden folgende bekannte Wirkstoffe als Vergleichsmittel verwendet:

A: N-Dodecyl-N,N-dimethyl-N-benzyl-ammoniumchlorid (Wallhäusser: Sterilisation-Desinfektion-Konservierung-Chemotherapie, Georg Thieme Verlag, Stuttgart, 1967, S. 222)

B: 1,3-Bis-(p-isobutylbenzyl)-imidazoliumchlorid (DE-B-1 216 487).

*Versuch 1*

Zur Ermittlung der Wirksamkeit der neuen Verbindungen gegenüber Bakterien werden zu je 5 ml steigender Verdünnungen der Wirkstoffe 5 ml doppelt konzentrierte Nähbouillon in sterile Reagenzgläser gegeben und vermischt. Durch Zugabe von einem

Tropfen einer 1:10 verdünnten 16 Stunden alten Bouillon-Kultur der Bakterien-Art Escherichia coli werden dann die Röhrchen beimpft und 24 Stunden bei 37°C bebrütet. Nach dieser Zeit werden Proben aus den Röhrchen auf Bakteriennährböden übertragen und diese ebenfalls 24 Stunden lang bei 37°C bebrütet. Diejenige Verdünnungsstufe, bei welcher nach dem Übertragen einer Probe auf den Nährboden keine Bakterienentwicklung mehr erfolgt, wird als Abtötungswert angegeben.

Das Ergebnis des Versuches zeigt, dass beispielsweise die Verbindungen 2, 3, 4, 5, 9, 10, 11 und 12 gegen Escherichia eine bessere bakterizide Wirkung haben (beispielsweise Verdünnung 12:1 Millionen Gew.-Teile) als der bekannte Wirkstoff B (beispielsweise 100:1 Million).

*Versuch 2*

Zur Prüfung der Wirksamkeit gegenüber Pilzen werden die Wirkstoffe einer für das Wachstum der Pilze Aspergillus niger, Oidium lactis bzw. Candida albicans in Mengen von 100, 50, 25, 12, 6 und 3 Gewichtsteilen pro Million Teile Nährlösung zugesetzt. Jeweils 10 ml Nährlösungs-Wirkstoffgemisch werden in sterile Reagenzgläser gegeben und mit einem Tropfen einer Sporensuspension beimpft, die $10^6$ Konidien bzw. Zellen enthält. Nach 120stündiger Bebrütung werden aus denjenigen Röhrchen, die kein sichtbares Pilzwachstum zeigen, Proben entnommen und auf Pilznährböden übertragen. Es wird die Verdünnungsstufe festgestellt, bei welcher nach dem Übertragen einer Probe auf den Nährboden kein Wachstum der Pilze mehr erfolgt.

Das Ergebnis des Versuches zeigt, dass beispielsweise die Verbindungen 1, 2, 3, 4, 5, 9, 10, 11 und 12 eine bessere fungizide Wirkung (beispielsweise 12:1 Million) haben als die bekannten Wirkstoffe A und B (beispielsweise 25:1 Million).

*Versuch 3*

Zur Prüfung der Wirksamkeit gegenüber Grünalgen werden die Wirkstoffe einer die Vermehrung der einzelligen Grünalge Chlorella vulgaris begünstigenden, phosphatreichen Nährlösung in Mengen von 10, 7,5, 5, 2,5 und 1 Gewichtsteilen pro Million Teile Nährlösung zugesetzt. Jeweils 100 ml Nährlösungs-Wirkstoffgemisch sowie nur Nährlösung (Kontrolle) werden in 300 ml-Glaskolben gegeben. Die Nährlösung wird vor dem Wirkstoffzusatz mit einer Suspension der Alge Chlorella vulgaris beimpft; die Zelldichte wird auf $10^6$ Zellen/ml Nährlösung eingestellt. Nach zehntägiger Aufstellung der Versuchsansätze bei Raumtemperatur und Lichtzutritt wird die Wirksamkeit beurteilt.

Das Ergebnis des Versuches zeigt, dass beispielsweise die Verbindungen 2 und 3 eine bessere algizide Wirkung haben (beispielsweise 2,5:1 Million) als die bekannten Wirkstoffe A und B (beispielsweise 5:1 Million).

**Patentansprüche für die Vertragsstaaten:**
BE, CH, DE, FR, GB, IT, LI, NL, SE

1. Substituiertes Benzylimidazoliumsalz der Formel

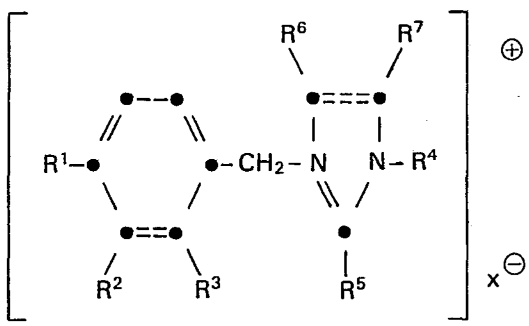

in der $R^1$, $R^2$ und $R^3$ gleich oder verschieden sind und Wasserstoff, einen $C_1$-$C_4$-Alkylrest oder Halogen, $R^4$ einen Alkylrest mit 8 bis 14 Kohlenstoffatomen, Benzyl, halogen- bzw. alkylsubstituiertes Benzyl, $R^5$ Wasserstoff oder einen Alkylrest mit 1 bis 8 Kohlenstoffatomen, Phenyl, Benzyl, $R^6$ Alkyl mit 1 bis 4 C-Atomen oder Halogen, $R^7$ Alkyl mit 1 bis 4 C-Atomen oder Halogen und X einen Säurerest bedeuten.

2. Verbindung gemäss Anspruch 1, dadurch gekennzeichnet, dass $R^1$ Wasserstoff oder Chlor, $R^2$ Wasserstoff oder Chlor, $R^3$ Methyl, $R^4$ Decanyl oder Dodecanyl, $R^5$ Wasserstoff, $R^6$ Methyl, $R^7$ Chlor und X Chlorid bedeuten.

3. Mikrobizid enthaltend ein Benzylimidazoliumsalz gemäss Anspruch 1.

4. Mikrobizid enthaltend ein Benzylimidazoliumsalz gemäss Anspruch 2.

5. Mikrobizid enthaltend einen festen oder flüssigen Trägerstoff und ein Benzylimidazoliumsalz gemäss Anspruch 1.

6. Verfahren zur Herstellung eines Mikrobizids, dadurch gekennzeichnet, dass man einen festen oder flüssigen Trägerstoff vermischt mit einem Benzylimidazoliumsalz gemäss Anspruch 1.

7. Verfahren zur Bekämpfung von Mikroben, dadurch gekennzeichnet, dass man die Mikroben oder die vor Mikrobenbefall zu schützenden Gegenstände behandelt mit einem Benzylimidazoliumsalz gemäss Anspruch 1.

8. 4-Methyl-5-chlorimidazol.

**Patentansprüche für den Vertragsstaat:** AT

1. Mikrobizid enthaltend ein Benzylimidazoliumsalz der Formel

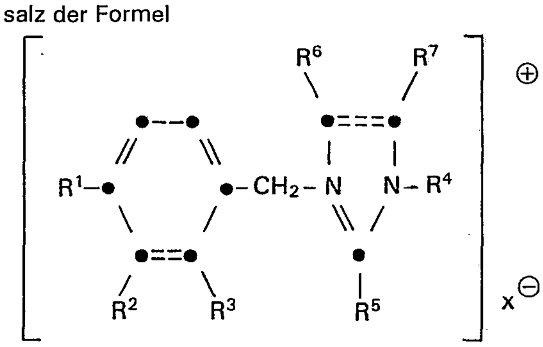

in der $R^1$, $R^2$ und $R^3$ gleich oder verschieden sind und Wasserstoff, einen $C_1$-$C_4$-Alkylrest oder Halogen, $R^4$ einen Alkylrest mit 8 bis 14 Kohlenstoffatomen, Benzyl, halogen- bzw. alkylsubstituiertes Benzyl, $R^5$ Wasserstoff oder einen Alkylrest mit 1 bis 8 Kohlenstoffatomen, Phenyl, Benzyl, $R^6$ Alkyl mit 1 bis 4 C-Atomen oder Halogen, $R^7$ Alkyl mit 1 bis 4 C-Atomen oder Halogen und X einen Säurerest bedeuten.

2. Mikrobizid, dadurch gekennzeichnet, dass $R^1$ Wasserstoff oder Chlor, $R^2$ Wasserstoff oder Chlor, $R^3$ Methyl, $R^4$ Decanyl oder Dodecanyl, $R^5$ Wasserstoff, $R^6$ Methyl, $R^7$ Chlor und X Chlorid bedeuten.

3. Mikrobizid enthaltend einen festen oder flüssigen Trägerstoff und ein Benzylimidazoliumsalz wie in Anspruch 1 definiert.

4. Verfahren zur Herstellung eines Mikrobizids, dadurch gekennzeichnet, dass man einen festen oder flüssigen Trägerstoff vermischt mit einem Benzylimidazoliumsalz wie in Anspruch 1 definiert.

5. Verfahren zur Bekämpfung von Mikroben, dadurch gekennzeichnet, dass man die Mikroben oder die vor Mikrobenbefall zu schützenden Gegenstände behandelt mit einem Benzylimidazoliumsalz wie in Anspruch 1 definiert.

**Claims for the Contracting States:**
BE, CH, DE, FR, GB, IT, LI, NL, SE

1. A substituted benzylimidazolium salt of the formula

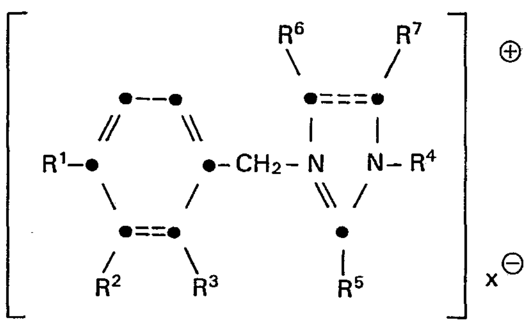

where $R^1$, $R^2$ and $R^3$ are identical or different and are each hydrogen, $C_1$-$C_4$-alkyl or halogen, $R^4$ is alkyl of 8 to 14 carbon atoms or benzyl which is unsubstituted or substituted by halogen or alkyl, $R^5$ is hydrogen, alkyl of 1 to 8 carbon atoms, phenyl or benzyl, $R^6$ is alkyl of 1 to 4 carbon atoms or halogen, $R^7$ is alkyl of 1 to 4 carbon atoms or halogen and X is an acid radical.

2. A compound as claimed in claim 1, wherein $R^1$ is hydrogen or chlorine, $R^2$ is hydrogen or chlorine, $R^3$ is methyl, $R^4$ is decanyl or dodecanyl, $R^5$ is hydrogen, $R^6$ is methyl, $R^7$ is chlorine and X is chloride.

3. A microbicide which contains a benzylimidazolium salt as claimed in claim 1.

4. A microbicide containing a benzylimidazolium salt as claimed in claim 2.

5. A microbicide which contains a solid or liquid carrier and a benzylimidazolium salt as claimed in claim 1.

6. A process for the preparation of a microbicide, wherein a solid or liquid carrier is mixed with a benzylimidazolium salt as claimed in claim 1.

7. A process for combating microbes, wherein the microbes, or the articles to be protected from attack by microbes, are treated with a benzylimidazolium salt as claimed in claim 1.

8. 4-methyl-5-chloro-imidazole.

**Claims for the Contracting State:** AT

1. A microbicide containing a benzylimidazolium salt of the formula

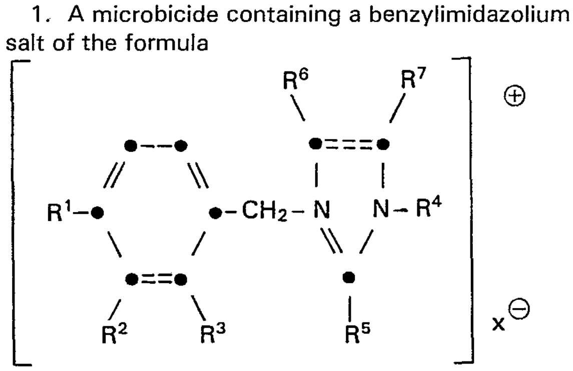

where $R^1$, $R^2$ and $R^3$ are identical or different and are each hydrogen, $C_1$-$C_4$-alkyl or halogen, $R^4$ is alkyl of 8 to 14 carbon atoms or benzyl which is unsubstituted or substituted by halogen or alkyl, $R^5$ is hydrogen, alkyl of 1 to 8 carbon atoms, phenyl or benzyl, $R^6$ is alkyl of 1 to 4 carbon atoms or halogen, $R^7$ is alkyl of 1 to 4 carbon atoms or halogen and X is an acid radical.

2. A microbicide, wherein $R^1$ is hydrogen or chlorine, $R^2$ is hydrogen or chlorine, $R^3$ is methyl, $R^4$ is decanyl or dodecanyl, $R^5$ is hydrogen, $R^6$ is methyl, $R^7$ is chlorine and X is chloride.

3. A microbicide which contains a solid or liquid carrier and a benzylimidazolium salt as defined in claim 1.

4. A process for the preparation of a microbicide, wherein a solid or liquid carrier is mixed with a benzylimidazolium salt as defined in claim 1.

5. A process for combating microbes, wherein the microbes, or the articles to be protected from attack by microbes are treated with a benzylimidazolium salt as defined in claim 1.

**Revendications pour les Etats contractants:**
BE, CH, DE, FR, GB, IT, LI, NL, SE

1. Sel de benzyl-imidazolium substitué de la formule

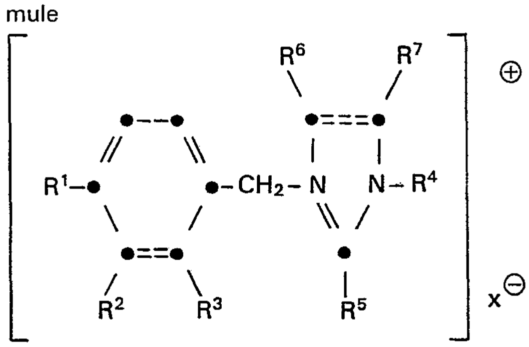

dans laquelle $R^1$, $R^2$ et $R^3$, qui peuvent être identiques ou différents, désignent chacun un atome d'hydrogène ou d'halogène ou un radical alkyle en $C_1$ à $C_4$, $R^4$ représente un groupe alkyle en $C_8$ à $C_{14}$, benzyle ou benzyle halo- ou alkyl-substitué, $R^5$ désigne un atome d'hydrogène, un radical alkyle en $C_1$ à $C_8$ ou un groupe benzyle ou phényle, $R^6$ désigne un radical alkyle en $C_1$ à $C_4$ ou un atome d'halogène et $R^7$ un radical alkyle en $C_1$ à $C_4$ ou un atome d'halogène et X représente le reste d'un acide.

2. Sel suivant la revendication 1, caractérisé en ce que $R^1$ = H ou Cl, $R^2$ = H ou Cl, $R^3$ = méthyle, $R^4$ = décanyle ou dodécanyle, $R^5$ = H, $R^6$ = méthyle, $R^7$ = Cl et X désigne un chlorure.

3. Composition microbicide, contenant un sel de benzyl-imidazolium suivant la revendication 1.

4. Composition microbicide, contenant un sel de benzyl-imidazolium suivant la revendication 2.

5. Composition microbicide, contenant un sel de benzyl-imidazolium suivant la revendication 1 dans une matière support solide ou un véhicule liquide.

6. Procédé de préparation d'une composition microbicide, caractérisé en ce que l'on incorpore un sel de benzyl-imidazolium suivant la revendication 1 à une matière solide ou un véhicule liquide.

7. Procédé de lutte contre les microbes, caractérisé en ce que l'on traite les microbes ou les objets à protéger des microbes avec un sel de benzyl-imidazolium suivant la revendication 1.

8. Le méthyl-4 chloro-5 imidazole.

**Revendications pour l'Etat contractant:** AT

1. Composition microbicide, contenant un sel de benzyl-imidazolium de la formule

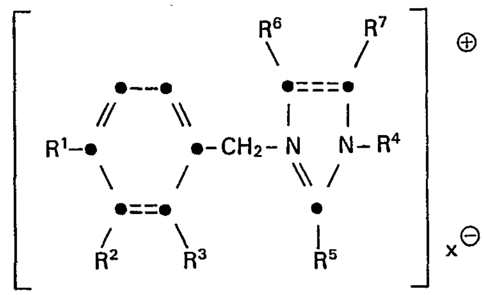

dans laquelle $R^1$, $R^2$ et $R^3$, qui peuvent être identiques ou différents, désignent chacun un atome d'hydrogène ou d'halogène ou un radical alkyle en $C_1$ à $C_4$, $R^4$ représente un groupe alkyle en $C_8$ à $C_{14}$, benzyle ou benzyle halo- ou alkyl-substitué, $R^5$ désigne un atome d'hydrogène, un radical alkyle en $C_1$ à $C_8$ ou un groupe benzyle ou phényle, $R^6$ désigne un radical alkyle en $C_1$ à $C_4$ ou un atome d'halogène et $R^7$ un radical alkyle en $C_1$ à $C_4$ ou un atome d'halogène et X représente le reste d'un acide.

2. Composition microbicide, caractérisée en ce que $R^1$ = H ou Cl, $R^2$ = H ou Cl, $R^3$ = méthyle, $R^4$ = décanyle ou dodécanyle, $R^5$ = H, $R^6$ = méthyle, $R^7$ = Cl et X désigne un chlorure.

3. Composition microbicide, contenant un sel de benzyl-imidazolium tel que défini dans la revendication 1 dans une matière support solide ou un véhicule liquide.

4. Procédé de préparation d'une composition microbicide, caractérisé en ce que l'on mélange un sel de benzyl-imidazolium tel que défini dans la revendication 1 à une matière support solide ou un véhicule liquide.

5. Procédé de lutte contre des microbes, caractérisé en ce que l'on traite les microbes ou les objets à protéger des microbes avec un sel de benzyl-imidazolium tel que défini dans la revendication 1.